# EUROPÄISCHE PATENTSCHRIFT

(11) **EP 1 845 080 B1**
(45) Veröffentlichungstag und Bekanntmachung des Hinweises auf die Patenterteilung: **26.03.2014**
(21) Anmeldenummer: 07075104.5
(22) Anmeldetag: 06.02.2007
(51) Int. Cl.: C07C 209/84, C07C 211/46

(54) **Verfahren zur Herstellung von Anilin**
Method for manufacturing aniline
Procédé de fabrication d'aniline

(30) Priorität: 18.02.2006 DE 102006007619
(43) Veröffentlichungstag der Anmeldung: 17.10.2007
(73) Patentinhaber: Bayer Intellectual Property GmbH, 40789 Monheim (DE)
(72) Erfinder: Dugal, Markus, 47906 Kempen (DE); Gehlen, Franz-Ulrich, 47839 Krefeld (DE); Wershofen, Stefan, 41065 Mönchengladbach (DE); Lago, Andre, Caojing Shanghai 201506 (CN); Lehner, Peter, 40878 Ratingen (DE); Bäcker, Werner, 51688 Wipperfürth (DE); Marotz, Benie, 40227 Düsseldorf (DE); Brinkschulte, Horst, 56290 Mörsdorf (DE)
(74) Vertreter: BIP Patents

(56) Entgegenhaltungen:
- US-A- 5 981 795
- DATABASE WPI Week 199704 Derwent Publications Ltd., London, GB; AN 1997-037989 XP002439113 -& JP 08 295654 A (SUMITOMO CHEM CO LTD) 12. November 1996 (1996-11-12)

## Beschreibung

Die Erfindung betrifft ein Verfahren zur Herstellung und Aufreinigung von Anilin durch Extraktion von Roh-Anilin mit wässriger Alkalimetallhydroxid-Lösung (Alkalilauge), wobei die Konzentration der eingesetzten Alkalilauge und die Temperatur so eingestellt werden, dass bei der Phasentrennung die wässrige Phase die untere Phase darstellt.

Anilin ist wichtiges Zwischenprodukt z.B. zur Herstellung von Methylendiphenyldiisocyanat (MDI) und wird im großtechnischen Maßstab in der Regel durch katalytische Hydrierung von Nitrobenzol mit Wasserstoff hergestellt (siehe z.B. DE-OS 2 201 528, DE-OS 3 414 714, US 3 136 818, EP 0 696 573 und EP 0 696 574). Bei dieser Reaktion werden neben dem Zielprodukt Anilin auch Nebenkomponenten wie z.B. Phenole oder Aminophenole gebildet, die vor einer weiteren Anwendung des Anilins in Folgeprozessen durch Destillation entfernt werden müssen. Insbesondere die Trennung von Phenol und Anilin stellt aufgrund der eng zusammen liegenden Siedepunkte für die Destillationstechnik eine große Herausforderung dar, was sich in der Verwendung langer Destillationskolonnen mit großer Trennstufenzahl und hoher Rücklaufverhältnisse mit entsprechend hohem Investitions- und Energieaufwand widerspiegelt.

Die Anmeldung JP-A-08-295654 beschreibt als Alternative zur Entfernung von phenolischen Verbindungen aus Anilin eine Extraktion mit verdünnter wässriger Natronlauge (oder Kalilauge), wodurch das Phenol als Natriumphenolat großenteils in die wässrige Phase transferiert wird, die durch die anschließende Phasentrennung als obere Phase abgetrennt wird. Dabei wird die Einstellung der Natronlaugekonzentration in einem Bereich von < 0,7 Gew.-% als notwendig angeführt, um eine Phasenumkehr und somit Probleme bei der Phasentrennung zu vermeiden. Für eine effektive Reduzierung des Phenolgehaltes ist ein molares Verhältnis NaOH : Phenol im Bereich von 3-100:1 erforderlich.

Nachteilig bei diesem Verfahren ist die Limitierung auf stark verdünnte wässrige Alkalihydroxidlösungen < 0,7 Gew.-% zur Vermeidung von Phasentrennproblemen und Phasenumkehr, da hierdurch bei einem gegebenen erforderlichen molaren Alkali:Phenol-Verhältnis die Menge an Alkali-Phenolat-haltigem Abwasser relativ groß werden kann, was ökologische und ökonomische Nachteile mit sich bringt.

Die Aufgabe der vorliegenden Erfindung ist daher, ein einfaches und wirtschaftliches Verfahren zur Aufreinigung von Anilin, das durch katalytische Hydrierung von Nitrobenzol hergestellt wurde, zur Verfügung zu stellen, bei dem auf die aufwendige Destillation verzichtet werden kann und bei dem gleichzeitig die Größe der Abwasserströme reduziert werden kann.

Die Erfindung betrifft ein Verfahren zur Herstellung von Anilin, bei dem
a) Roh-Anilin durch Hydrierung von Nitrobenzol in Gegenwart eines Katalysators hergestellt wird, und
b) das Roh-Anilin mit wässriger Alkalimetallhydroxid-Lösung extrahiert und anschließend die wässrige und die organische Phase voneinander getrennt werden, wobei die Konzentration der eingesetzten Alkalimetallhydroxid-Lösung und die Temperatur während der Extraktion so eingestellt werden, dass bei der Trennung der wässrigen und der organischen Phase die wässrige Phase die untere Phase darstellt.

Das eingesetzte Roh-Anilin kann aus allen technisch üblichen Verfahren zur Hydrierung von Nitrobenzol stammen. Bevorzugt wird die Hydrierung von Nitrobenzol in der Gasphase an ortsfesten, heterogenen Trägerkatalysatoren, wie z.B. Pd auf Aluminiumoxid oder Kohleträgern, in Festbettreaktoren bei einem Druck von 2-50 bar und einer Temperatur im Bereich von 250-500°C unter adiabatischen Bedingungen in Kreisgasfahrweise, d.h. unter Rückführung von während der Hydrierung nicht umgesetztem Wasserstoff, durchgeführt (siehe EP-A-0 696 573 und EP-A-0 696 574).

Dabei wird eine Alkalimetallhydroxid-Lösung in einem Konzentrationsbereich von > 0,7 Gew.-% an Alkalimetallhydroxid, bezogen auf das Gewicht der Alkalimetallhydroxid-Lösung, eingesetzt, um zu erreichen, dass bei der Trennung der wässrigen und der organischen Phase die wässrige Phase die untere Phase darstellt. Durch Einstellung höherer Temperaturen wird das Vorliegen der wässrigen Phase als untere Phase zusätzlich begünstigt.

Durch das erfindungsgemäße Vorgehen kann also sichergestellt werden, dass keine Phasentrennprobleme oder Phasenumkehr bei der in der Extraktion enthaltenen Phasentrennung auftreten. Denn bei dem erfindungsgemäßen Verfahren ist die wässrige Phase stets die untere Phase, eine Phasenumkehr während der Separation, beispielsweise in dem Phasentrennbehälter, tritt daher nicht auf.

Bevorzugt wird als Alkalimetallhydroxid-Lösung Natriumhydroxid-Lösung oder Kaliumhydroxid-Lösung eingesetzt. Besonders bevorzugt wird Natriumhydroxid-Lösung eingesetzt. Grundsätzlich können aber alle Alkalimetallhydroxid-Lösungen eingesetzt werden. Auch der Einsatz von Erdalkalimetallhydroxiden oder anderen wasserlöslichen basischen Verbindungen wie z.B. Alkali- oder Erdalkalimetallcarbonaten oder Hydrogencarbonaten ist grundsätzlich denkbar.

Der bevorzugte Konzentrationsbereich der eingesetzten Alkalimetallhydroxid-Lösung liegt zwischen 0,71 und 35 Gew.-%, an Alkalimetallhydroxid bevorzugt zwischen 0,75 und 10 Gew.-%, bezogen auf das Gewicht der Alkalimetallhydroxid-Lösung.

Die Temperatur bei der Extraktion liegt je nach Alkalimetallhydroxid-Konzentration bevorzugt im Bereich zwischen 20°C und 140°C, besonders bevorzugt zwischen 30°C und 100°C, ganz besonders bevorzugt zwischen 50 °C und 95°C. Bevorzugt liegt die Temperatur während der Phasentrennung, die ein Bestandteil der Extraktion ist, in den gleichen Bereichen.

Die Wahl der geeigneten Kombination der Konzentration der Alkalimetallhydroxid-Lösung und der Temperatur während der Extraktion richtet sich neben der Erreichung einer unten liegenden wässrigen Phase bei der Phasentrennung nach den jeweilig relevanten verfahrenstechnischen und ökonomischen Kriterien. So kann einerseits zur Begrenzung der Wasserlöslichkeit des Anilins eine Minimierung der Temperatur sinnvoll sein, andererseits kann es verfahrenstechnisch von Vorteil sein, das Rohanilin bei höherer Temperatur nach der Reaktion zu kondensieren und bei gleicher Temperatur dann auch zu extrahieren. Weiterhin kann eine zu hohe Konzentration der Alkalimetallhydroxid-Lösung zu einer verminderten Extraktionseffizienz und verlängerten Trennzeiten führen, wenn das Organik/Wasser-Verhältnis dadurch zu groß wird und eine zu niedrige Konzentration der Alkalimetallhydroxid-Lösung zu den genannten Nachteilen einer zu großen Abwassermenge führt.

Das zur Herstellung der wässrigen Alkalimetallhydroxid-Lösung verwendete Wasser wird vorzugsweise ganz oder teilweise dem Reaktionswasser aus der Hydrierreaktion von Nitrobenzol entnommen, wodurch sich eine zusätzliche Verringerung der gesamten Abwasserfracht des Anilin-Herstellverfahrens erreichen lässt. Es kann jedoch auch Wasser aus jeder beliebigen anderen Quelle eingesetzt werden. Die verdünnte Alkalimetallhydroxid-Lösung, die zur Extraktion eingesetzt wird, wird in der Regel durch Zudosierung einer konzentrierten Alkalimetallhydroxid-Lösung zum Einsatzwasser erzeugt, wobei die konzentrierte Alkalimetallhydroxid-Lösung das Alkalimetallhydroxid, z.B. NaOH oder KOH, in bevorzugten Konzentrationen von 2 bis 50 Gew.-% Alkalimetallhydroxid, bezogen auf das Gewicht der Alkalihydroxid-Lösung, enthält.

Für die Extraktion können alle dem Fachmann bekannten Methoden und Apparaturen, wie z.B. Mixer-Settler oder Extraktionskolonnen eingesetzt werden. Die Extraktion kann einstufig oder mehrstufig im Gleich- oder Gegenstrom erfolgen. In einer bevorzugten Ausführungsform wird eine zweistufige Gegenstrom Mixer-Settler-Apparatur für die Extraktion eingesetzt. Zur Absenkung der notwendigen Trenn- und Verweilzeiten können die Abscheider mit Koaleszierhilfen wie z.B. Gestricken, Platten oder Füllkörpern versehen werden.

Das in dem erfindungsgemäßen Verfahren erhältliche gereinigte Anilin enthält bevorzugt weniger als 0,01 Gew.-%, besonders bevorzugt weniger als 0,005 Gew.-% an phenolischen Verbindungen in der Summe, bezogen auf das Gewicht des Anilins. Dabei sind unter phenolischen Verbindungen neben Phenol und Phenolat auch diejenigen Benzol-Derivate zusammengefasst, die zusätzlich zur OH-Funktion auch weitere funktionelle Gruppen tragen, wie. z.B. Aminophenole.

Weitere Aufarbeitungsschritte, wie z.B. Destillations- oder Waschstufen können der Extraktion mit Alkalimetallhydroxid-Lösungen zur Erreichung noch höherer Reinheitsgrade des Anilins vor- und/oder nachgeschaltet werden, sind aber nicht zwingend erforderlich. Die nach- oder vorgeschalteten Wasch- und/oder Destillationsschritte können in allen dem Fachmann geläufigen Varianten ausgestaltet und unter verschiedensten Bedingungen betrieben werden. So kann eine Destillation z.B. in einer oder mehreren Glockenboden- oder Packungskolonnen, aber auch in Trennwandkolonnen erfolgen. Dabei können Leichtsieder und Schwersiederabtrennung in verschiedenen Kolonnen, aber auch gemeinsam in einer Kolonne unter Seitenstromentnahme des Anilins erfolgen.

Die destillative Aufarbeitung des von phenolischen Verbindungen weitgehend gereinigten Rohanilins, kann nach verschiedenen Methoden unter Einstellung einer großen Bandbreite an Bedingungen erfolgen. Dabei kann die Destillation ein- oder mehrstufig in verschiedenen Kolonnentypen, bevorzugt in konventionellen Rektifikationskolonnen oder in Ausführungen als Trennwandkolonnen und mit den verschiedenen Einbauten wie z.B. Sieb-, Ventil- oder auch Glockenböden, Füllkörpern oder Packungen erfolgen. Auch andere Ausführungen sind möglich. Die Betriebsparameter Kopfdruck und Rücklaufverhältnis sind immer in Abhängigkeit von der Zusammensetzung des Rohanilins, der Spezifikation/Reinheit des gereinigten Anilins (Reinanilins) und den zur Verfügung stehenden Trennstufen zu wählen. Die Abtrennung von Leichtsiedern, wie z.B. Wasser, Benzol, Cyclohexan, Cyclohexylamin, Cyclohexanon und Schwersiedern wie z.B. Phenol, Alkaliphenolate, Aminophenole, Alkaliaminophenolate, Phenylendiamine, Diphenylamin etc. kann dabei separat in verschiedenen Kolonnen erfolgen oder alternativ, in einer bevorzugten Ausführungsform, kombiniert in einer Kolonne unter Kopfentnahme der Leichtsieder, Sumpfentnahme der Schwersieder und Seitenstromentnahme des Reinanilins. Die destillative Reinigung des von phenolischen Verbindungen weitgehend gereinigten Roh-Anilins erfolgt in einer bevorzugten Ausführungsform in einer Seitenstromkolonne, besonders bevorzugt in einer Trennwandkolonne, unter Entnahme der Leichtsieder am Kopf, Entnahme der Schwersieder am Sumpf und Entnahme des Reinanilins im Seitenstrom. Weiterhin kann das Sumpfprodukt der Schwersiederabtrennung optional in einer Reststoffkolonne weiter eingeengt werden, um den Anilin-Verlust zu minimieren.

Der Zulauf des von phenolischen Verbindungen weitgehend gereinigten Rohanilins in die Destillationskolonne kann an einer beliebigen Stelle der Kolonne erfolgen, bevorzugt erfolgt der Zulauf jedoch entsprechend dem Konzentrationsprofil des Anilins in der Destillationskolonne in der Kolonnenmitte oder in der unteren Kolonnenhälfte. Die Kolonne kann einen Abtriebs- und/oder einen Verstärkerteil besitzen. Die Zulauftemperatur in die Kolonne, sowie die Sumpftemperatur, Kopfdruck und Rücklaufverhältnis sind einstellbar und können der Trennaufgabe, sowie den qualitativen, betrieblichen und ökonomischen Erfordernissen angepasst werden. Die Temperatur am Kopf der Kolonne stellt sich entsprechend der gewählten Voreinstellungen der genannten Parameter und der Zusammensetzung der flüssigen Phase und der Dampfphase in der Kolonne ein. Bevorzugte Bedingungen für Betriebsparameter der Destillationskolonne sind absolute Drücke von 10 bis 1000 mbar, besonders bevorzugt 10 bis 500 mbar und Rücklaufverhältnisse von 0,1 bis 3, besonders bevorzugt von 0,3 bis 0,8.

In einer besonderen Ausführungsform der Erfindung erfolgt der Zulauf bzw. die Zudosierung des Rohanilins in eine Leichtsiederkolonne, in der die Leichtsieder inklusive des Wassers über Kopf der Kolonne abgetrennt werden. Das im Sumpf anfallende Gemisch enthaltend Anilin und Hochsieder wird anschließend einem weiteren Destillationsschritt (Schwersiederabtrennung oder Reindestillation) zugeführt. Optional folgt schließlich eine Aufkonzentrierung des Sumpfes in einer Reststoffkolonne, wobei das in der Reststoffkolonne über Kopf gewonnene Anilin wieder in die Kolonne der Schwersiederabtrennung oder Reindestillation oder in die Leichtsiederkolonne bzw. die vorgeschaltete Phasentrennung zurückgeführt werden kann.

In einer weiteren besonderen Ausführungsform erfolgt der Zulauf des von phenolischen Verbindungen weitgehend gereinigten Roh-Anilins in eine kombinierte Leichtsieder- und Schwersiederkolonne (Seitenstromkolonne), wobei die Leichtsieder über Kopf, die Schwersieder als Sumpfphase und das Reinanilin als Seitenstrom abgeführt wird. Diese Seitenstromkolonne ist sowohl als konventionelle Kolonne (d.h. ohne Trennwand) als auch als Trennwandkolonne realisierbar. Diese Variante, bei der eine Seitenstromkolonne oder Trennwandkolonne eingesetzt wird, erfordert eine Phasentrennung der am Kopf entnommenen kondensierten Brüden, die im Wesentlichen das Azeotrop Wasser / Anilin und Leichtsieder enthalten. Wasser und in der wässrigen Phase gelöste Leichtsieder werden bevorzugt abgeführt, das Anilin wird bevorzugt zur Kolonne zurückgeführt.

Bevorzugt werden in dieser Ausführungsform des erfindungsgemäßen Verfahrens die am Kopf der Seitenstromkolonne entnommen Brüden in einer zweistufigen Kondensation kondensiert. Dabei kondensiert der erste Kondensator bevorzugt partiell die schwerer siedenden Komponenten der Brüden. In dem zweiten, nachgeschalteten Kondensator werden bevorzugt die durchgeschlagenen Leichtsieder kondensiert, die somit separat ausgeschleust werden können. Das Partialkondensat aus dem ersten Kondensator wird einer Phasentrennung zugeführt. Wasser und in der wässrigen Phase gelöste Leichtsieder werden bevorzugt abgeführt, das Anilin wird bevorzugt zur Kolonne zurückgeführt.

Das im Seitenstrom entnommene Rein-Anilin wird bevorzugt partiell als Rücklauf auf die Seitenstromkolonne unterhalb der Entnahmestelle des Seitenstroms aufgegeben. Die Seitenstromentnahme kann als Totalentnahme oder als partielle Entnahme realisiert werden. In beiden Fällen ist eine gezielte Einstellung des Rücklaufverhältnisses realisierbar.

Die für die Extraktion verwendete Alkalimetallhydroxid-Lösung kann nach der Extraktion, ggf. nach zusätzlicher Reinigung und/oder Aufkonzentrierung, rezykliert und wieder zur Extraktion verwendet werden. Alternativ kann die für die Extraktion verwendete Alkalimetallhydroxid-Lösung, ggf. nach zusätzlicher Reinigung, einem Abwasserstrom zugeführt werden, der beispielsweise nach anschließender Aufarbeitung einer Kläranlage zugeführt wird.

Das nach dem erfindungsgemäßen Verfahren erhaltene Anilin kann anschließend nach den aus dem Stand der Technik bekannten Verfahren mit Formaldehyd in Gegenwart eines sauren Katalysators zu Di- und Polyaminen der Diphenylmethanreihe umgesetzt werden. Die Di- und Polyaminen können anschließend nach den aus dem Stand der Technik bekannten Verfahren mit Phosgen zu den entsprechenden Di- und Polyisocyanaten der Diphenylmethareihe umgesetzt werden.

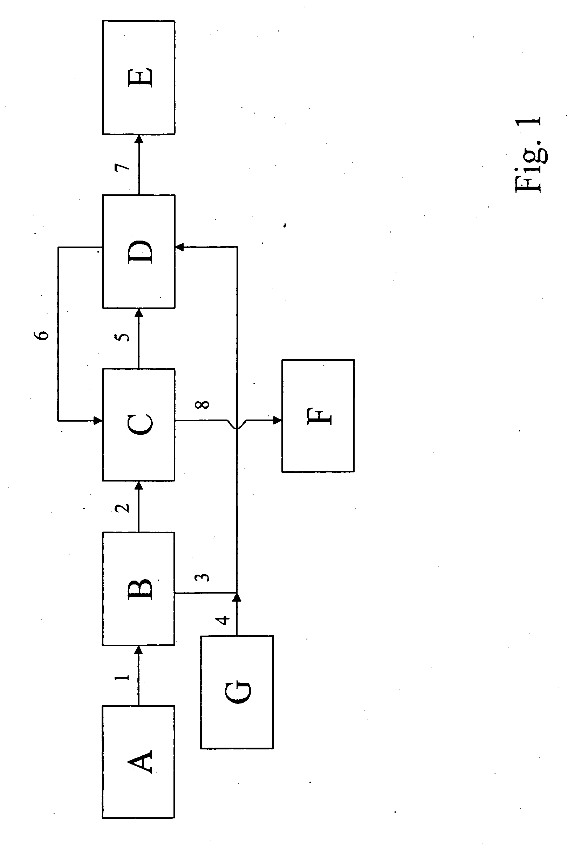

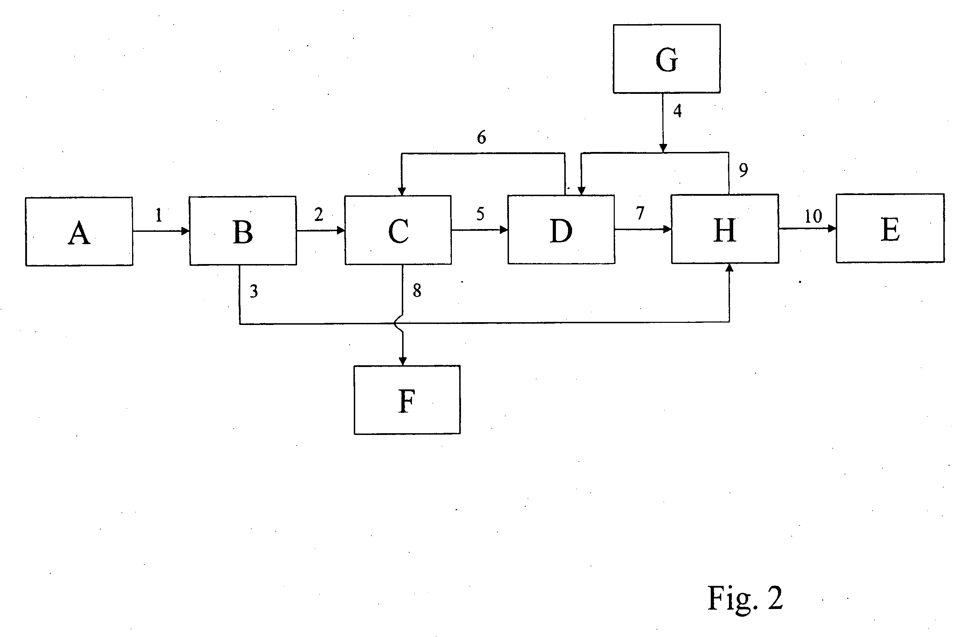

Die Figuren 1 und 2 zeigen in schematischer Darstellung bevorzugte Ausführungsformen des erfindungsgemäßen Verfahrens.

In Figur 1 ist eine bevorzugte Ausführungsform des erfindungsgemäßen Verfahrens dargestellt. Aus dem Reaktionsteil A einer Anlage zur Herstellung von Roh-Anilin wird das Gemisch 1 aus Rohanilin und Reaktionswasser in einen Phasentrenner B überführt. Nach Abtrennung der Wasserphase wird das Rohanilin 2 in eine erste Mixer-Settler-Extraktionsstufe C geleitet. Das Wasser 3 wird durch Zugabe von Natronlaugelösung 4 aus einem Vorratsbehälter G auf die gewünschte NaOH-Konzentration eingestellt und in eine zweite Mixer-Settler-Extraktionsstufe D geleitet. Das einmalig extrahierte Anilin 5 aus der ersten Extraktionsstufe C wird in die zweite Extraktionsstufe D geleitet, während die als untere Phase abgetrennte wässrige Natronlaugelösung 6 aus der zweiten Extraktionsstufe D im Gegenstrom in die erste Extraktionsstufe C überführt wird. Das zweimalig extrahierte Anilin 7 wird anschließend einer destillativen Aufarbeitungsstufe E zugeführt, die als untere Phase abgetrennte wässrige Alkalilösung 8 aus der ersten Mixer-Settler-Extraktionsstufe C wird einer Abwasseraufarbeitungstufe F zugeführt.

In Figur 2 ist eine alternative, ebenfalls bevorzugte Ausführungsform des erfindungsgemäßen Verfahrens dargestellt. Aus dem Reaktionsteil A einer Anlage zur Herstellung von Roh-Anilin wird das Gemisch 1 aus Rohanilin und Reaktionswasser in einen Phasentrenner B überführt. Nach Abtrennung der Wasserphase im Phasentrenner B wird das Rohanilin 2 in eine erste Mixer-Settler-Extraktionsstufe C geleitet. Das im Phasentrenner B abgetrennte Reaktionswasser 3 wird zunächst einer Waschstufe H zugeführt, in der das zweimalig mit NaOH-Lösung extrahierte Anilin 7 vor der Destillation E gewaschen wird. Das abgetrennte Wasser 9 aus der Waschstufe H wird mit Natronlauge 4 aus einem Vorlagebehälter G versetzt und in die zweite Extraktionsstufe D überführt. Das einmalig extrahierte Anilin 5 aus der ersten Extraktionsstufe C wird in die zweite Extraktionsstufe D geleitet, während die als untere Phase abgetrennte wässrige Natronlaugelösung 6 aus der zweiten Extraktionsstufe D im Gegenstrom in die erste Extraktionsstufe C überführt wird. Das zweimalig extrahierte Anilin 7 wird nach der Wäsche H einer destillativen Aufarbeitungsstufe E als Strom 10 zugeführt, die als untere Phase abgetrennte wässrige Alkalilösung 8 aus der ersten Mixer-Settler-Extraktionsstufe C wird einer Abwasseraufarbeitungstufe F zugeführt.

In einer weiteren Modifizierung der beschriebenen Ausführungsformen kann die wässrige Alkalilösung 8 im Kreis geführt und ggf. unter Ausschleusung einer Teilmenge und Ergänzung durch frische Alkalilösung wieder zur Extraktion in die zweite Mixer-Settler-Extraktionsstufe D überführt werden.

Alternativ kann die Extraktion bei beiden Fahrweisen auch einstufig oder mehr als zweistufig ausgeführt werden.

### Beispiele

Im Folgenden sind Beispiele für die Durchführung des erfindungsgemäßen Verfahrens gegeben. Die Analyse der Phenolgehalte in den nachfolgenden Beispielen erfolgt mittels Gaschromatographie (GC), die Analyse der Natrium-Gehalte erfolgt mittels Atomabsorptionsspektroskopie (AAS).

### Beispiel 1

In Beispiel 1 wird ein phenolhaltiges Rohanilin mittels des erfindungsgemäßen Verfahrens gereinigt und gereinigtes Anilin (Reinanilin) erhalten. Bei einem vordefinierten Gewichts-Verhältnis von organischer zu wässriger Phase von 4,9:1 wird mit 2,5 Gew.-% Natronlauge (2,5 Gew.% Na-OH bezogen auf das Gewicht der NaOH-Lösung) das im Rohanilin enthaltene Phenol durch eine zweistufige Gegenstromextraktion in Mixer-Settler Apparaten abgereichert. Dabei ist die wässrige Phase in den Phasentrenn-Behältern (Settler) die untere Phase. Die Betriebsparameter sind in Tabelle 1 zusammengefasst. Es wird eine Phenolabreicherung von 939 ppm auf 35 ppm erzielt (Tabelle 1).

**Tabelle 1**

| Temperatur °C | Rohanilin Phenol ppm | Zulaufmenge Anilinphase g/h | NaOH-Lösung g/h | NaOH-Lösung ml/h | NaOH-Konz. Gew.-% | Phasenverhältnis G.t. OP/WP | Molarer Überschuss x fach | Abl. Extr.. Phenol ppm |
|---|---|---|---|---|---|---|---|---|
| 90 | 939 | 1950 | 400 | 390 | 2,5 | 4,90 | 12,85 | 35 |

| | | | | | | | | |
|---|---|---|---|---|---|---|---|---|
| (Gew-% = Gewichts-%, Konz. = Konzentration, Phasenverh.= Phasenverhältnis, G.t. = Gewichtsteile, OP = Organische Phase, WP = Wässrige Phase, Abl. Extr. = Ablauf) | | | | | | | | |

In einer anschließenden Destillation in einer Seitenstromkolonne wird Reinanilin als Seitenstromprodukt abgezogen. Die Betriebsparameter und Phenolabreicherung sind in Tabelle 2 aufgeführt.

**Tabelle 2**

| Zullauf Feed kg/h | Zulauf Phenol ppm | R/E | Betriebsdruck mbar (abs) | Seitenstrom kg/h | Seitenstrom Phenolgehalt Ppm | LS/H S ppm | Wasser-konz. ppm | Kopfkondensat wässrige Phase g/h | Org. Phase g/h (Kreislauf) |
|---|---|---|---|---|---|---|---|---|---|
| 2,1 | 35 | 1,1 | 133 | 1,8 | 10 | 38/15 | 1000 | 206 | 2,7 |

| | | | | | | | | | |
|---|---|---|---|---|---|---|---|---|---|
| (R/E = Rücklaufverhältnis, LS = Leichtsieder, HS = Hochsieder, Wasserkonz. = Wasserkonzentration, org. = organische) | | | | | | | | | |

### Beispiel 2

Bei einem definierten Gewichts-Verhältnis von organischer zu wässriger Phase von 3,87:1 wird der Phenolgehalt im Rohanilin durch eine zweistufige Gegenstromextraktion in Mixer-Settler Apparaten mit 0,8 Gew.-% Natronlauge (0,8 Gew.% NaOH bezogen auf das Gewicht der NaOH-Lösung) von 388 ppm auf 26 ppm abgereichert. Die Betriebsparameter sind in Tabelle 3 zusammengefasst. Dabei ist die wässrige Phase in den Phasentrenn-Behältern (Settler) die untere Phase.

**Tabelle 3**

| Tempe ratur °C | Rohanlin Phenol ppm | Zulaufmenge Anilinphase g/h | NaOH-Lösung g/h | NaOH-Lösung ml/h | NaOH-Konz. Gew.-% | Phasenverhältnis G.t. OP/WP | Molarer Überschuss x fach | Abl. Extr.. Phenol ppm |
|---|---|---|---|---|---|---|---|---|
| 90 | 388 | 2420 | 624,7 | 620 | 0,8 | 3,87 | 12,52 | 26 |

In einer anschließenden Destillation in einer Seitenstromkolonne wird ein Reinanilin als Seitenstromprodukt abgezogen. Die Betriebsparameter und die erreichten Konzentrationen und Phenolabreicherung zeigt Tabelle 4:

**Tabelle 4**

| Zulauf Feed kg/h | Zulauf Phenol ppm | R/E | Betriebsdruck mbar (abs) | Seitenstrom kg/h | Seitenstrom Phenolgehalt ppm | LS/H S ppm | Wasser-konz. ppm | Kopfkondensat wässrige Phase g/h | Org. Phase g/h (Kreislauf) |
|---|---|---|---|---|---|---|---|---|---|
| 2,1 | 26 | 0,8 | 133 | 1,8 | 9 | 81/72 | 1100 | 190 | 2,3 |

### Beispiel 3

In Beispiel 3 werden 50 g eines phenolhaltiges Rohanilins durch eine zweistufige Kreuzstromextraktion in Schütteltrichtern bei 90°C und einem Verhältnis organischer zu wässriger Phase von 5,0:1 mit 1,5 Gew.-% NaOH-Lösung (1,5 Gew.% NaOH bezogen auf das Gewicht der NaOH-Lösung) extrahiert. Dabei ist die wässrige Phase in den Phasentrenn-Behältern (Settler) die untere Phase. Anschließend wird das erhaltene gereinigte Anilin einer Wasserwäsche zur Reduzierung des Rest-Na-Gehalts zugeführt. Der Phenolgehalt im Rohanilin wird dadurch von 494 ppm auf 50 ppm reduziert. Durch eine nachfolgende Wasserwäsche wird der Phenolgehalt von 50 ppm auf 40 ppm abgesenkt, der Na-Gehalt der organischen Phase sinkt von 27 ppm auf 9 ppm (siehe Tabelle 5). Die Betriebsparameter sind ebenfalls in Tabelle 5 zusammengefasst.

**Tabelle 5**

| | Rohanilin | Ablauf 1. Extraktionstufe | Ablauf 2. Extraktionsstufe | Ablauf Wasserwäsche |
|---|---|---|---|---|
| Phenol (ppm) | 494 | 140 | 50 | 40 |
| Na (ppm) | 0,9 | 72 | 27 | 9,4 |

## Patentansprüche

1. Verfahren zur Herstellung von Anilin, bei dem
a) Roh-Anilin durch Hydrierung von Nitrobenzol in Gegenwart eines Katalysators hergestellt wird, und
b) das Roh-Anilin mit wässriger Alkalimetallhydroxid-Lösung extrahiert und anschließend die wässrige und die organische Phase voneinander getrennt werden, wobei die Konzentration dereingesetzten Alkalimetallhydroxid-Lösung und die Temperatur während der Extraktion so eingestellt werden, dass bei derTrennung derwässrigen und der organischen Phase die wässrige Phase die untere Phase darstellt, wobei die Alkalimetallhydroxid-lösung das Alkalimetallhydroxid in Konzentrationen von > 0,7 Gew.%, bezogen auf das Gewicht der Alkalimetallhydroxidlösung, enthält.

2. Verfahren nach Anspruch 1 bei dem die Hydrierung des Nitrobenzols in der Gasphase unter adiabatischen Bedingungen in Festbettreaktoren und unter Rückführung von während der Hydrierung nicht umgesetztem Wasserstoff in Gegenwart eines Pd-haftigen Katalysators durchgeführt wird.

3. Verfahren nach einem der Ansprüche 1 oder 2, bei dem die Alkalimetallhydroxid-Lösung durch Verdünnung einer höher konzentrierten Alkalimetallhydroxid-Lösung mit Wasser hergestellt wird, wobei das Wasser zumindest teilweise bei der Hydrierung von Nitrobenzol anfällt.

4. Verfahren nach einem der Ansprüche 1 bis 3, bei dem als Alkalimetallhydroxid Natrium-und/oder Kaliumhydroxid eingesetzt wird.

5. Verfahren nach einem der Ansprüche 1 bis 4, bei dem die Alkalimetallhydroxid-Lösung das Alkalimetallhydroxid in Konzentrationen von zwischen 0,71 und 35 Gew.%, bezogen auf das Gewicht der Alkalimetallhydroxid-Lösung, enthält.

6. Verfahren nach einem der Ansprüche 1 bis 5, bei dem die Extraktion bei Temperaturen von 20°C bis 140°C durchgeführt wird.

7. Verfahren nach einem der Ansprüche 1 bis 6, bei dem die eingesetzte Alkalimetallhydroxid-Lösung nach der Extraktion und Phasentrennung gegebenenfalls gereinigt und aufkonzentriert und anschließend in die Extraktion zurückgeführt wird.

8. Verfahren nach einem der Ansprüche 1 bis 7, bei dem das Roh-Anilin vor der Extraktion und/oder das in der Extraktion erhaltene gereinigte Anilin in einer ein- oder mehrstufigen Destillation gereinigt wird.

9. Verfahren nach einem der Ansprüche 1 bis 8, bei dem das In der Extraktion erhaltene gereinigte Anilin in einer ein- oder mehrstufigen Wasserwäsche gereinigt wird.

10. Verfahren nach einem der Ansprüche 1 bis 9, bei dem das in der Extraktion erhaltene gereinigte Anilin in einer ein-oder mehrstufigen Wasserwäsche und einer daran anschießenden ein- oder mehrstufigen Destillation gereinigt wird.

11. Verfahren nach Anspruch 8, bei dem die Destillation des in der Extraktion erhaltenen gereinigten Anilins, ggf. nach einer ein- oder mehrstufigen Wasserwäsche, einstufig in einer Seitenstromkolonne durchgeführt wird, wobei die Leichtsieder über Kopf, die Schwersieder als Sumpfprodukt und das Reinanilin als Seitenstrom abgeführt wird.

12. Verfahren nach Anspruch 8, bei dem die Destillation des in der Extraktion erhaltenen gereinigten Anilins, ggf. nach einer ein- oder mehrstufigen Wasserwäsche, einstufig in einer Trennwandkolonne durchgeführt wird, wobei die Leichtsieder über Kopf, die Schwersieder als Sumpfprodukt und das Reinanilin als Seitenstrom abgeführt wird.

13. Verfahren nach einem der Ansprüche 11 oder 12, bei dem die in der Seitenstromkolonne oderTrennwandkolonne am Kopf abgezogenen Brüden in einer zweistufigen Kondensation kondensiert werden.

14. Verfahren zur Herstellung von Di- und Polyaminen der Diphenylmethanreihe, bei dem Anilin nach dem Verfahren gemäß Anspruch 1 hergestellt wird und anschließend das Anilin mit Formaldehyd in Gegenwart eines sauren Katalysators zu den Di- und Polyaminen umgesetzt wird.

15. Verfahren zur Herstellung von Di- und Polyisocyanaten der Diphenylmethanreihe, bei dem Anilin nach dem Verfahren gemäß Anspruch 1 hergestellt wird und anschließend das Anilin mit Formaldehyd in Gegenwart eines sauren Katalysators zu Di- und Polyaminen umgesetzt wird und anschließend die Di- und Polyamine mit Phosgen zu den Di- und Polyisocyanaten umgesetzt werden.

## Claims

1. Process for preparing aniline, wherein
a) crude aniline is prepared by hydrogenation of nitrobenzene in the presence of a catalyst and
b) the crude aniline is extracted with aqueous alkali metal hydroxide solution and the aqueous and organic phases are subsequently separated, where the concentration of the alkali metal hydroxide solution used and the temperature during the extraction are adjusted such that in the separation of the aqueous phase and the organic phase, the aqueous phase is the bottom phase, where the alkali metal hydroxide solution comprises the alkali metal hydroxide in concentrations of > 0.7 wt% based on the weight of the alkali metal hydroxide solution.

2. Process according to Claim 1, wherein the hydrogenation of the nitrobenzene is carried out in the gas phase under adiabatic conditions in fixed-bed reactors and with recycling of hydrogen that has not reacted during the hydrogenation, in the presence of a Pd-containing catalyst.

3. Process according to either of Claims 1 and 2, wherein the alkali metal hydroxide solution is prepared by diluting a more highly concentrated alkali metal hydroxide solution with water, the water at least partly being formed during the hydrogenation of nitrobenzene.

4. Process according to any one of Claims 1 to 3, wherein sodium hydroxide and/or potassium hydroxide are used as alkali metal hydroxide.

5. Process according to any one of Claims 1 to 4, wherein the alkali metal hydroxide solution comprises the alkali metal hydroxide in concentrations of between 0.71 and 35 wt% based on the weight of the alkali metal hydroxide solution.

6. Process according to any one of Claims 1 to 5, wherein the extraction is carried out at temperatures in the range from 20 to 140°C.

7. Process according to any one of Claims 1 to 6, wherein, following the extraction and phase separation, the alkali metal hydroxide solution used is optionally purified and concentrated and subsequently recycled into the extraction.

8. Process according to any one of Claims 1 to 7, wherein the crude aniline is purified in a single- or multi-stage distillation prior to the extraction and/or the purified aniline obtained in the extraction is purified in a single- or multi-stage distillation.

9. Process according to any one of Claims 1 to 8, wherein the purified aniline obtained in the extraction is purified in a single- or multi-stage water wash.

10. Process according to any one of Claims 1 to 9, wherein the purified aniline obtained in the extraction is purified in a single- or multi-stage water wash and a subsequent single- or multi-stage distillation.

11. Process according to Claim 8, wherein the distillation of the purified aniline obtained in the extraction is optionally preceded by a single- or multi-stage water wash and is carried out in a single stage in a side stream column, the low boilers being removed overhead, the high boilers being removed as bottom product and the pure aniline being removed as side stream.

12. Process according to Claim 8, wherein the distillation of the purified aniline obtained in the extraction is optionally preceded by a single- or multi-stage water wash and is carried out in a single stage in a dividing wall column, the low boilers being removed overhead, the high boilers being removed as bottom product and the pure aniline being removed as side stream.

13. Process according to either of Claims 11 and 12, wherein the vapours withdrawn overhead in the side stream column or in the dividing wall column are condensed in a two-stage condensation.

14. Process for preparing di- and polyamines of the diphenylmethane series, wherein aniline is prepared by the process according to Claim 1 and the aniline is subsequently reacted with formaldehyde in the presence of an acidic catalyst to form the di- and polyamines.

15. Process for preparing di- and polyisocyanates of the diphenylmethane series, wherein aniline is prepared by the process according to Claim 1 and the aniline is subsequently reacted with formaldehyde in the presence of an acidic catalyst to form the di- and polyamines and the di- and polyamines are subsequently reacted with phosgene to form the di- and polyisocyanates.

## Revendications

1. Procédé pour la production d'aniline, dans lequel
a) on prépare de l'aniline brute par hydrogénation de nitrobenzène en présence d'un catalyseur, et
b) on extrait l'aniline brute à l'aide d'une solution aqueuse d'un hydroxyde de métal alcalin et ensuite on sépare l'une de l'autre la phase aqueuse et la phase organique, en ajustant pendant l'extraction la concentration de la solution d'hydroxyde de métal alcalin utilisée et la température de manière que lors de la séparation de la phase aqueuse et de la phase organique la phase aqueuse représente la phase inférieure, la solution d'hydroxyde de métal alcalin contenant l'hydroxyde de métal alcalin à des concentrations de > 0,7 % en poids, par rapport au poids de la solution d'hydroxyde de métal alcalin.

2. Procédé selon la revendication 1, dans lequel l'hydrogénation du nitrobenzène est effectuée dans la phase gazeuse dans des conditions adiabatiques, dans des réacteurs à lit fixe, en présence d'un catalyseur contenant du Pd et avec recyclage de l'hydrogène n'ayant pas réagi pendant l'hydrogénation.

3. Procédé selon l'une quelconque des revendications 1 et 2, dans lequel on prépare la solution d'hydroxyde de métal alcalin par dilution avec de l'eau d'une solution plus concentrée d'hydroxyde de métal alcalin, l'eau étant produite au moins en partie dans l'hydrogénation du nitrobenzène.

4. Procédé selon l'une quelconque des revendications 1 à 3, dans lequel on utilise comme hydroxyde de métal alcalin l'hydroxyde de sodium et/ou de potassium.

5. Procédé selon l'une quelconque des revendications 1 à 4, dans lequel la solution d'hydroxyde de métal alcalin contient l'hydroxyde de métal alcalin à des concentrations comprises entre 0,71 et 35 % en poids, par rapport au poids de la solution d'hydroxyde de métal alcalin.

6. Procédé selon l'une quelconque des revendications 1 à 5, dans lequel on effectue l'extraction à des températures de 20 °C à 140 °C.

7. Procédé selon l'une quelconque des revendications 1 à 6, dans lequel après l'extraction et la séparation des phases la solution d'hydroxyde de métal alcalin utilisée est éventuellement purifiée et concentrée et ensuite renvoyée dans l'extraction.

8. Procédé selon l'une quelconque des revendications 1 à 7, dans lequel on purifie dans une distillation en un ou plusieurs stades l'aniline brute avant l'extraction et/ou l'aniline purifiée obtenue dans l'extraction.

9. Procédé selon l'une quelconque des revendications 1 à 8, dans lequel l'aniline purifiée, obtenue dans l'extraction, est purifiée dans un lavage à l'eau en un ou plusieurs stades.

10. Procédé selon l'une quelconque des revendications 1 à 9, dans lequel l'aniline purifiée, obtenue dans l'extraction, est purifiée dans un lavage à l'eau en un ou plusieurs stades et une distillation en un ou plusieurs stades y faisant suite.

11. Procédé selon la revendication 8, dans lequel la distillation de l'aniline purifiée, obtenue dans l'extraction, est effectuée, éventuellement après un lavage à l'eau en un ou plusieurs stades, en un stade dans une colonne à courant latéral, les composants à bas point d'ébullition étant évacués par la tête, les composants à haut point d'ébullition étant évacués en tant que produit de pied et l'aniline pure étant déchargée en tant que courant latéral.

12. Procédé selon la revendication 8, dans lequel la distillation de l'aniline purifiée, obtenue dans l'extraction, est effectuée, éventuellement après un lavage à l'eau en un ou plusieurs stades, en un stade dans une colonne à paroi de séparation, les composants à bas point d'ébullition étant évacués par la tête, les composants à haut point d'ébullition étant évacués en tant que produit de pied et l'aniline pure étant déchargée en tant que courant latéral.

13. Procédé selon l'une quelconque des revendications 11 et 12, dans lequel les vapeurs évacuées à la tête dans la colonne à courant latéral ou la colonne à paroi de séparation sont condensées dans une condensation en deux stades.

14. Procédé pour la production de di- et polyamines de la série du diphénylméthane, dans lequel on produit de l'aniline conformément à un procédé selon la revendication 1 et ensuite on fait réagir l'aniline avec du formaldéhyde en présence d'un catalyseur acide, pour aboutir aux di- et polyamines.

15. Procédé pour la production de di- et polyisocyanates de la série du diphénylméthane, dans lequel on produit de l'aniline conformément au procédé selon la revendication 1 et ensuite on fait réagir l'aniline avec du formaldéhyde en présence d'un catalyseur acide, pour aboutir à des di- et polyamines et ensuite on fait réagir les di- et polyamines avec du phosgène, pour aboutir aux di- et polyisocyanates.
